# EUROPÄISCHE PATENTSCHRIFT

(11) **EP 1 636 135 B1**
(45) Veröffentlichungstag und Bekanntmachung des Hinweises auf die Patenterteilung: **11.05.2011**
(21) Anmeldenummer: 04740344.9
(22) Anmeldetag: 25.06.2004
(51) Int. Cl.: C01G 1/02, C01G 19/00, C01G 23/00, C01F 17/00, G01N 27/12, G01N 33/00

(54) **MISCHMETALLOXIDE UND IHRE VERWENDUNG IN CO2-SENSOREN**
MIXED METAL OXIDES AND USE THEREOF IN CO2 SENSORS
OXYDES METALLIQUES MIXTES ET LEUR UTILISATION DANS DES DETECTEURS DE CO2

(30) Priorität: 25.06.2003 DE 10329626
(43) Veröffentlichungstag der Anmeldung: 22.03.2006
(73) Patentinhaber: ItN Nanovation AG, 66117 Saarbrücken (DE)
(72) Erfinder: FABER, Stefan, 66133 Saarbrücken (DE); MATHUR, Sanjay, 66125 Saarbrücken (DE); NONNINGER, Ralph, 66129 Saarbrücken (DE); MEYER, Frank, 66111 Saarbrücken (DE); VEITH, Michael, 66386 St. Ingbert (DE); SHEN, Hao, 66125 Saarbrücken (DE)
(74) Vertreter: Patentanwälte Ruff, Wilhelm, Beier, Dauster & Partner
(86) Internationale Anmeldenummer: PCT/EP2004/006938
(87) Internationale Veröffentlichungsnummer: WO 2004/113229

(56) Entgegenhaltungen:
- EP-A- 0 263 394
- DE-A- 4 437 692
- GB-A- 2 206 571
- US-B1- 6 533 966
- WEI Q ET AL: "Giant capacitance effect and physical model of nano crystalline <formula><roman>CuO–BaTiO</roman><in f>3</inf></formula> semiconductor as a <formula><roman>CO</roman><inf>2</inf></fo rmula> gas sensor" JOURNAL OF APPLIED PHYSICS, AMERICAN INSTITUTE OF PHYSICS. NEW YORK, US, Bd. 88, Nr. 8, 15. Oktober 2000 (2000-10-15), Seiten 4818-4824, XP012051821 ISSN: 0021-8979 in der Anmeldung erwähnt
- VEITH M ET AL: "SOL-GEL SYNTHESIS OF NANO-SCALED BATIO3, BAZRO3 AND BATI0.5ZR0.5O3 OXIDES VIA SINGLE-SOURCE ALKOXIDE PRECURSORS AND SEMI-ALKOXIDE ROUTES" JOURNAL OF SOL-GEL SCIENCE AND TECHNOLOGY, KLUWER ACADEMIC PUBLISHERS, DORDRECHT, NL, Bd. 17, Nr. 2, 1. Februar 2000 (2000-02-01), Seiten 145-158, XP001020298 ISSN: 0928-0707

## Beschreibung

Die Erfindung betrifft neue Mischmetalloxide sowie deren Herstellung und Verwendung sowie einen neuen CO₂-Sensor auf Basis dieser Mischmetalloxide.

CO₂-Sensoren dienen zum Brand- oder Explosionsschutz, zur Prozeßüberwachung in industriellen Anlagen oder werden in Sensorarrays als "Chemische Nasen" eingesetzt. Weiterhin finden CO₂-Sensoren zur Messung der Raumluftqualität, von Auto- oder Industrieabgasen oder im Biomonitoringbereich (Gärprozesse, Fouling, Fermentierung, Atmung etc.) oder in Klimaeinrichtungen ein immer breiteres Anwendungsfeld.

Für nicht brennbare, d. h. nicht oxidierbare Gase wie CO₂, gibt es derzeit zwei verschiedene Meßprinzipien:

Zum einen sind dies optische Methoden mittels NDIR (nicht dispersive Infrarotabsorption). Diese Methode detektiert die CO₂-Absorptionsbande bei 4,27 µm und zeichnet sich durch eine hohe Empfindlichkeit aus. Sie ist sehr aufwendig, da eine komplizierte Optik in Verbindung mit Präzisionsmechanik (Spektrometer) benötigt wird. Solche Sensoren werden auch in Zukunft keine Verbreitung in Massenmärkten, in denen ein einfacher und kostengünstiger Aufbau wichtig ist, finden.

Zum anderen handelt es sich um elektrochemische Sensoren, bei denen ein Potentialunterschied zwischen Meß- und gekapselter Referenzelektrode bei der Adsorption von Gasmolekülen detektiert wird. Solche Sensoren sind aufwendig, leiden unter einer langen Ansprechzeit (bis zu 30 Sekunden), einer Querempfindlichkeit auf Luftfeuchte und einem störungsanfälligen Aufbau. Einen Spezialfall solcher elektrochemischer CO₂-Sensoren findet man auf Basis natriumionenleitender Festelektrolyten mit einer Alkalicarbonatelektrode. Diese sind als NASICON-Sensoren (Natrium Super-Ionic Conductor) bekannt. Solche Systeme müssen wegen ihrer Feuchtigkeitsempfindlichkeit weitgehend gekapselt sein und haben eine lange Ansprechzeit, sind aber langzeitstabil und empfindlich.

Da beide Verfahren sehr kostenintensiv sind, muß ein anderes Meßprinzip zur CO₂-Detektion gesucht werden. Geeignet erscheinen vor allem halbleitende Materialien, die in der Lage sind, CO₂-Moleküle reversibel zu adsorbieren und bei einer Gasadsorption mit einer detektierbaren Widerstandsänderung zu reagieren. Ein solches Verfahren sollte sich bei Applikation des Halbleiters als dünne Schicht auf einem Trägermaterial preisgünstig realisieren lassen.

Als halbleitendes, gassensitives Material wurde beispielsweise dotiertes SnO₂ untersucht [Tamaki, Akiyama, Xu, Chemistry Letters (1990), 1243 ]. Dieses ist jedoch zur selektiven CO₂-Detektion nicht geeignet, da die Nachweisgrenze zu hoch liegt und die Querempfindlichkeiten zu oxidierbaren Gasen (vor allem zu CO und H₂) nicht unterdrückt werden können [Delabie, Honore, Lenaerts et al., Sensors and Actuators B, (1997), 44, 446]. Darüber hinaus ist die Homogenität der Dotierung nicht zu gewährleisten, was zu nicht reproduzierbaren Messergebnissen führt [Kim, Yoon, Park et al., Sensors and Actuators B (2000), 62, 61].

Weitere für Gassensoren geeignete Zinn-Verbindungen werden in der EP 263 394 beschrieben. Ein Gassensor basierend auf BaSnO₃ kann demnach hergestellt werden, indem Barium-Stannat aus Wasser ausgefällt wird und das Präzipitat anschließend thermisch behandelt wird.

Wei et al. [J. Appl. Phys. (2000), 88, 4818] beschäftigten sich mit CuO-BaTiO₃-Verbindungen und deren Eignung als CO₂-Sensoren, wobei die CuO-BaTiO₃-Verbindungen durch Vermischen von BaCO₃-Pulver und TiO₂-Pulver in equimolarem Verhältnis und anschließendes Sintern bei 1200 °C gewonnen wurden.

Ein Kohlendioxid-Sensor auf Basis eines Materials aus Kupferoxid und Barium-Titanat wird in der DE 44 37 692 A1 beschrieben. Dieses Material wird aufwendig durch Kugelmahlen synthetisiert, was große Nachteile mit sich bringt: Neben dem großen Aufwand ist durch Partikelaggregation verfahrensbedingt die effektive Oberfläche des Materials durch Agglomeration der einzelnen Kristallite stark eingeschränkt. Weiterhin sind die Dotierungen nicht homogen verteilt und tendieren dazu, beim Sintern an die Korngrenzen zu wandern. Darüber hinaus lassen sich mit den erwähnten Verfahren keine monodispersen Partikel herstellen, was dazu führt, daß größere neben kleineren Partikeln vorliegen, die aufgrund ihrer verschieden großen Oberfläche unterschiedlich empfindlich auf CO₂ reagieren.

Ein Gassensor für Sauerstoff auf Basis von Gd-dotiertem BaCeO₃ wird in der GB 2 206 571 A beschrieben. Die Herstellung dieses Materials erfolgt ebenfalls durch Mahlen aus den entsprechenden Oxiden, wobei die durch das Mahlen erhaltenen Partikel Korngrößen im Bereich zwischen 5 bis 40 µm aufweisen.

Insbesondere die Herstellung von Nanopartikeln durch Mahlvorgänge weist weitere grundsätzliche Nachteile auf. So findet man den Abrieb der Mahlbecher und Mahlkugeln im resultierenden Nanomaterial wieder, der Zeitbedarf ist sehr groß (bis zu mehreren Wochen), die Partikelgrößenverteilung ist sehr breit und das erhaltene Material weist üblicherweise in hohem Grad Defekte, Gitterspannungen und Gitterfehler auf. Auf diese Weise hergestellte Materialien können katalytische Eigenschaften aufweisen oder als Elektronenleiter verwendet werden. Als Sensormaterial für einen Gassensor sind sie hingegen unbrauchbar, da dafür ein weitestgehend defektfreies, homogenes, molekulardotiertes Material ohne Gitterfeh ler benötigt wird.

Die vorstehend erwähnten Nachteile führen dazu, daß aufgrund der bekannten Herstellungsverfahren und Materialien die Sensitivität und Selektivität von Gassensoren, insbesondere von CO₂-Sensoren, sehr stark verbesserungsbedürftig sind. Ein Sensor auf Basis bekannter Materialien ist für eine kommerzielle Anwendung ungeeignet.

Der Erfindung liegt somit die Aufgabe zugrunde, neue Materialien zu entwickeln. Diese sollen insbesondere in einer einfachen und kostengünstigen Meßanordnung als Gassensor mit hoher Empfindlichkeit und Spezifizität eingesetzt werden.

Diese Aufgabe wird gelöst durch Mischmetalloxide mit den Merkmalen das Anspruchs 11.

Das erfindungsgemäße Verfahren zeichnet sich dadurch aus, dass mit Hilfe der sogenannten Single-Source-Precursortechnik ein Mischmetallalkoxid hergestellt wird, das in der Stöchiometrie und der Struktur auf das herzustellende Mischmetalloxid abgestellt ist, und dieses Mischmetalloxid nach einem Dotierungsschritt zu dem Mischmetalloxid hydrolysiert wird.

Ebenso Gegenstand der vorliegenden Erfindung ist die Verwendung solcher Mischmetalloxide oder von Mischungen daraus zur Detektion von Gasen, vorzugsweise zur Detektion nicht brennbarer Gase.

Daneben ist auch ein Sensor zur Detektion von CO₂ mit den Merkmalen das Anspruchs 1 Gegenstand der vorliegenden Erfindung. Zur Offenbarung der Erfindung soll an dieser Stelle ausdrücklich auf die Formulierungen der einzelnen Ansprüche Bezug genommen und verwiesen werden. In diesem Zusammenhang sollen die in den Ansprüchen verwendeten Begriffe wie folgt noch näher erläutert werden.

Die Numerierung der Gruppen des Periodensystems, die in den Ansprüchen angegeben ist, erfolgt in der IUPAC-Version, bei der die einzelnen Gruppen des Periodensystems einfach durchnumeriert werden.

Der Begriff "nanoskatig" soll aussagen, daß sich die mittlere Teilchengröße der Mischmetalloxid-Partikel weit im sub-µm-Bereich befindet. Dabei soll sich diese Teilchengröße auf das einzelne Partikel im nicht-agglomerierten Zustand beziehen. Nanoskalige Partikel lagern sich aufgrund ihrer hohen Oberflächenenergien häufig zusammen und bilden auf diese Weise Agglomerate oder Teilchentrauben, die eine größere Teilchengröße vortäuschen, als das einzelne Teilchen tatsächlich besitzt. Die Größenangaben bei der Erfindung beziehen sich dementsprechend, soweit möglich, auf die mittlere Teilchengröße eines einzelnen Teilchens/Partikels, das in diesem Zusammenhang auch als "Primärteitchen" bezeichnet werden kann. Die mittlere Partikelgröße der erfindungsgemäßen Mischmetalloxide beträgt weniger als 100 nm, insbesondere weniger als 50 nm.

Bei den erfindungsgemäßen Mischmetalloxiden handelt es sich vorzugsweise um halbleitende Materialien, wobei die Eigenschaften von Halbleitern aus dem Stand der Technik bekannt sind. Solche halbleitenden Materialien sind (dotiert oder undotiert) auf vielfältige Weise einsetzbar, insbesondere als Gassensoren, beispielsweise zur Detektion von CO₂.

Das erfindungsgemäße Verfahren ist auf die Herstellung der Mischmetalloxide durch die sogenannte Single-Source-Precursortechnik beschränkt. Bei verschiedenen erfindungsgemäßen Mischmetalloxiden wird es durchaus möglich sein, diese Substanzen aus zwei oder mehreren nebeneinander getrennt vorliegenden Ausgangsverbindungen, wie beispielsweise Alkoxiden, herzustellen. Das Vorliegen einer einzelnen solchen Ausgangsverbindung, beispielsweise eines solchen Alkoxids als Single-Source ist in solchen Fällen nicht zwingend erforderlich.

Es zeigte sich, daß insbesondere zur CO₂-Detektion dotierte Mischmetalloxide (Perowskite) sowie Metall-Metalloxid-Komposite mit einer Dotierung geeignet sind. Diese Substanzen können jedoch nicht ohne weiteres hergestellt werden, da etablierte Methoden wie Fällung oder klassische Sol-Gel-Chemie häufig nicht verwendet können. Es mangelt entweder an geeigneten reaktiven Vorstufen, oder man hat es vor allem im Falle der Sol-Gel-Prozesses mit individuellen Precursoren (Alkoxiden) zu tun, die sich durch stark unterschiedliche Reaktivitäten und Hydrolysegeschwindigkeiten auszeichnen. Einfache Metallalkoxide können über metallorganische Synthesen hergestellt werden und sind seit einiger Zeit bekannt. So kann z. B. Bariumisopropanolat durch Kochen von Bariummetall in wasserfreiem Isopropanol unter Inertgasatmosphäre hergestellt werden. Andere Metallalkoholate sind wie die Propylate von Titan oder Zirkon zwar bereits großtechnisch verfügbar. Eine Mischung aus solchen Alkoxiden mit anderen Alkoxiden kann nach Hydrolyse und Aufarbeitung zu einem ungleichmäßigen Gefüge führen, das beispielsweise als CO₂-Sensor nicht geeignet ist. Für andere Zusammensetzungen sind die Vorstufen kommerziell nicht verfügbar. Weiterhin ist für eine optimale Leistungscharakteristik eine homogene Dotierung im vorzugsweise niedrigen Atom-%-Bereich nötig, was durch diese Methoden nicht erreicht werden kann.

Es konnte nun gefunden werden, daß sich insbesondere nanoskalige dotierte Bariumcerate und Bariumstannatverbindungen als CO₂-sensitive Materialien eignen, bei denen es jedoch essentiell ist, auf molekularer Ebene maßzuschneidern, was diese Patentschrift zeigt.

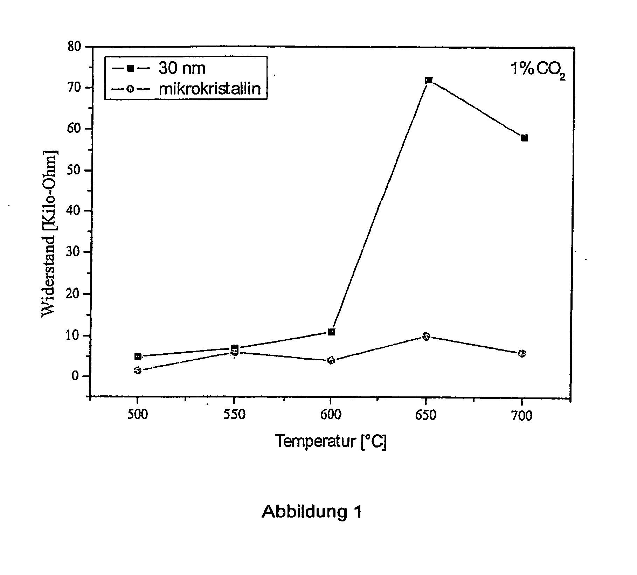

Überraschenderweise wurde nun ermittelt, daß insbesondere die für das CVD-Verfahren bekannte Single-Source-Precursortechnik [R.C. Mehrotra, Chemtracts: Org. Chem, (1990) 2, 338 oder Sing and Mehrotra, Z. Anorg. Allg. Chemie, (1984), 512, 221] dazu geeignet ist, ebensolche CO₂-sensitive Materialien mit hoher Homogenität sowohl einfach als auch kostengünstig herzustellen. Diese Materialien weisen die auf molekularer Ebene erwünschte homogene Dotierung und Vermischung auf, wozu hydrolysierbare komplexe Metallalkoxidverbindungen als molekulare Template verwendet werden. Dieses Single-Source-Verfahren erzielt extrem reine und vorzugsweise im Atom-%-Bereich von 0,01 bis 10 % dotierte Verbindungen, die ausgezeichnete Gassensoreigenschaften, wie in Abbildung 1 dargelegt, besitzen. Darüber hinaus lassen sich sehr kleine und monodisperse Kristallitgrößen und somit große Oberflächen realisieren, was für die Empfindlichkeit eines Sensors sehr wichtig ist.

In einer besonderen Ausführungsform der Erfindung wird das verwendete Alkoxid, das kommerziell nicht erhältlich ist, selbst synthetisiert.

Erfindungsgemäß wurde als CO₂-sensitives Material vorzugsweise dotiertes BaCeO₃ identifiziert. Es gelang hier zum erstenmal die Herstellung eines homogen mit Cu dotierten BaCeO₃ mit CO₂-sensitiven und selektiven Eigenschaften, was weit über den Stand der Technik hinausgeht.

Das so erhaltene Material kann über dem Fachmann bekannte Sieboder Tampondruckverfahren als Schicht auf einem Sensorsubstrat, z. B. Aluminiumoxid (Al₂O₃), abgeschieden oder in kommerzielle Sensorplattformen (z. B. von Heraeus) implementiert werden. Wird dann der (elektrische) Widerstand des nanoskaligen dotierten BaCeO₃ in CO₂-Atmosphäre in Abhängigkeit von der Temperatur gemessen, erkennt man in Abbildung 1 überraschenderweise bei 600 °C einen sprunghaften Anstieg im Signal des Sensors. Bei einer mikrokristallinen BaCeO₃-Vergleichprobe mit einer sehr viel geringeren Oberfläche ist ein solcher sprunghafter Anstieg nicht zu erkennen. Dies bedeutet, daß insbesondere ein nanokristallines Material (hier: Teilchengröße 30 nm) CO₂-sensitive Eigenschaften besitzt.

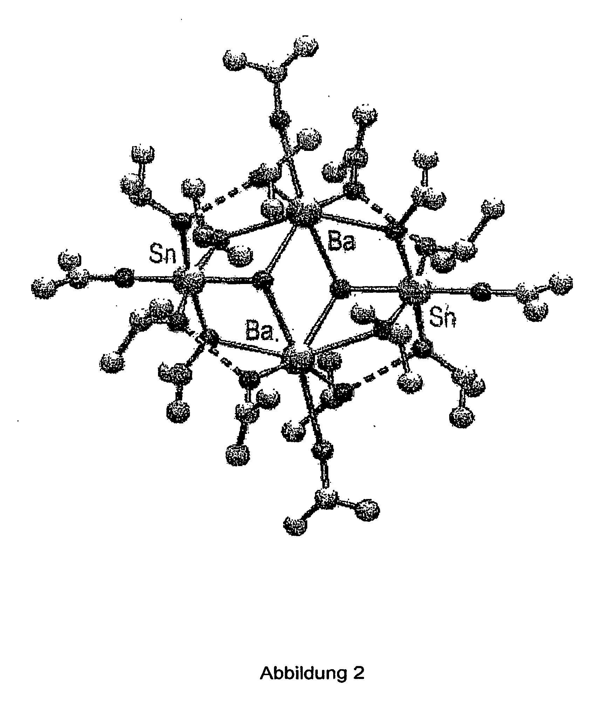

Ausgangssubstanzen für das Sensormaterial sind Mischmetallalkoxide, die in einem Molekül das Metall-Metall-Verhältnis auf molekularer Ebene (Ba:Ce hier 1:1) des nach Hydrolyse resultierenden Oxids bereits vorgegeben haben und durch Sauerstoffbrücken miteinander verknüpft sind. Beispiel (Abbildung 2: BaSn(OiPr)₈) als Precursor für BaSnO₃). iPr steht für Isopropyl. Auch kompliziertere Zusammensetzungen wurden realisiert (Ba(Ti,Ce)(OR)₈ für Ba(Ti_{0.5}ce_{0.5})O₃). R steht für Alkyl, vorzugsweise Isopropyl. Exemplarisch kann man die Struktur an einem Precursor für BaSnO₃ in Abbildung 2 erkennen.

Von großem Vorteil ist, im Precursormolekül die Stöchiometrie und die Struktur der nach der Hydrolyse resultierenden Verbindung vorzugeben. Hierzu verbindet das nach der Hydrolyse der Alkoxide gebildete dreidimensionale Netzwerk der phasenbildenden Elemente alle relevanten Atome (Sauerstoff bzw. Metall) in der richtigen Anordnung chemisch miteinander. Dieses Gerüst liefert das Fundament für die Nanopartikel, die bereits bei niedrigen Temperaturen ausgebildet werden. Das nach Aufarbeitung erhaltene CO₂-sensitive Material ist auf mesoskopischer Ebene homogen dotiert und phasenrein, und besitzt eine nahezu monodisperse Partikelverteilung im nm-Bereich. Das Material kann optional thermisch nachbehandelt, d. h. kristallisiert werden. Die Stoffe fallen entweder bereits bei der Hydrolyse kristallin an oder werden schonend hydrothermal im Hochdruckautoklaven kristallisiert. Gegenüber einer Kalzinierung besitzt die Hydrothermaltechnik den Vorteil, eine Partikelagglomeration zu vermeiden und die Oberfläche im reaktiven, d. h. modifizierbaren Zustand zu belassen.

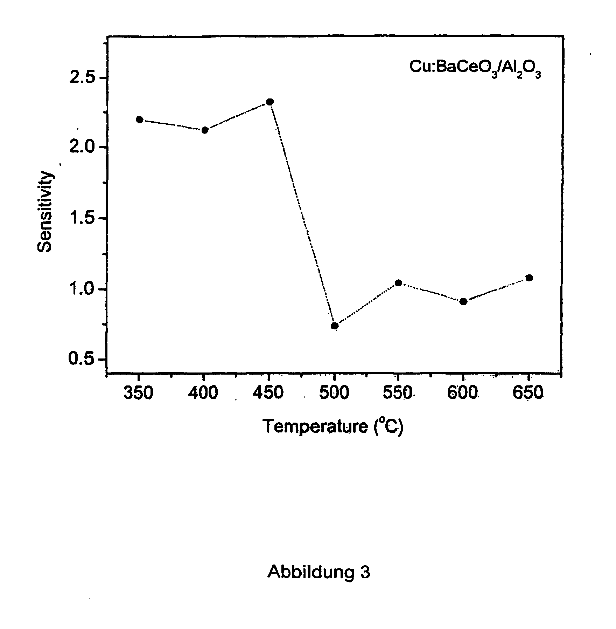

Aber auch aus einer Nachbehandlung durch Kalzinierung resultieren Materialien mit hervorragenden CO₂-sensitiven Eigenschaften. Abbildung 3 beispielsweise zeigt die temperaturabhängige Gassensitivität von mit 5 % Kupfer dotiertem BaCeO₃. Das Material war über ein spinund/oder dip-coating Verfahren auf Aluminiumoxid abgeschieden worden und liegt als dünner Film vor. Das Material mit einer mittleren Partikelgröße von 20 nm war bei 1000 °C hitzebehandelt worden. Es zeigte im Bereich niedriger Temperaturen (350 °C bis 450 °C) eine deutlich höhere CO₂-Sensitivität als im Bereich höherer Temperaturen (500 bis 650 °C).

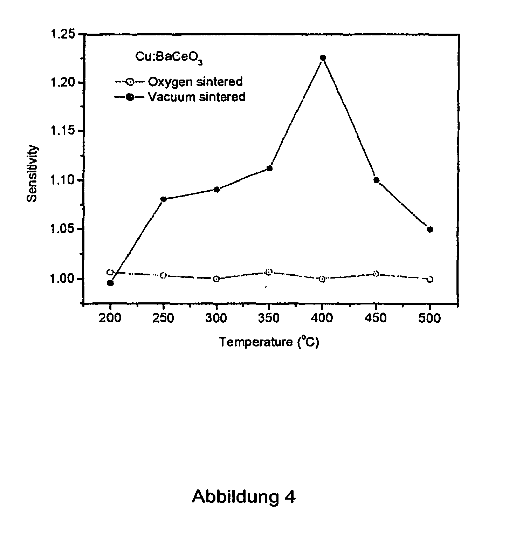

Herstellungsverfahren und auch thermische Nachbehandlung können eine große Rolle in Bezug auf die CO₂-Sensitivität der resultierenden Materialien spielen. Durch die thermische Nachbehandlung können die Oberflächeneigenschaften der Materialien beeinflusst werden. Im Falle der Nachbehandlung durch Kalzinierung wurde überraschenderweise gefunden, daß eine Durchführung der thermischen Nachbehandlung im Vakuum zu einem starken Anstieg der CO₂-Sensitivität führen kann. Abbildung 4 zeigt ebenfalls die temperaturabhängige Gassensitivität von mit 5 % Kupfer dotiertem, nanoskaligem BaCeO₃. Aufgetragen ist auch hier die Sensitivität gegen die Temperatur (°C), einmal für einen Sensor aus Material, das im Vakuum gesintert wurde, einmal für ein Material, das unter Anwesenheit von Sauerstoff gesintert wurde. Im Vergleich ist deutlich zu sehen, dass das Sintern im Vakuum zu deutlich höherer Sensitivität führt als das vergleichbare Sintern unter Anwesenheit von Sauerstoff.

Das beschriebene Verfahren ist preisgünstig, aufskalierbar und reproduzierbar. Es können verschiedene Metalle gezielt in eine Trägermatrix eindotiert werden, wozu kein anderes in der Literatur bekanntes Verfahren geeignet ist. Der Sensor ist extrem empfindlich, spezifisch (keine Querempfindlichkeit auf Wasser oder CO) und hat eine sehr niedrige Betriebstemperatur, die sich positiv auf die Stabilität und damit die Betriebsdauer auswirkt.

### 1. Beispiel für BaCeO₃ mit Kupferdotierung

8,506 g Ba(O^{t}Bu)₂ (0.03mol) (^{t}Bu steht für tert.-Butyl.) werden in absolutem PrⁱOH (200 ml) (Prⁱ bzw. ⁱPr steht für Isopropyl.) suspendiert, und eine stöchiometrische Menge Ce(OⁱPr)₄ (9.827g, 0.03mol) wird langsam unter Rühren unter Schutzgasatmosphäre dazugegeben. Die trübe Mischung wird 6 h bis zum Erhalt einer klaren Lösung gerührt. Zu diesem Zeitpunkt hat sich ein gemischtes Ba-Ce-Alkoxid, genaugenommen Ba-Ce[(O^{t}Bu)₂(OⁱPr)₄], gebildet, das im Gegensatz zu den ursprünglichen Alkoholaten in PrⁱOH löslich ist. Anschließend kann eine Dotierung mit einer Kupferquelle wie CuCl₂ in Alkohol oder einem Cu-Alkoxid in der gewünschten Stöchiometrie erfolgen. Dazu werden 0,255 g CuCl₂ (5 Mol-%) in 10 ml Isopropanol dazugegeben und für 2 h stark gerührt. Man erhält ein transparentes grünes Sol. Das dotierte Ba-Ce-Alkoxidsol wird mit einer stöchiometrischen Menge einer 1,0 molaren Wasserlösung in Isopropanol unter heftigem Rühren vermengt. Das weiterhin klare Sol wird bei 45 °C Badtemperatur am Rotationsverdampfer eingeengt. Das eingeengte BaCeO₃-Sol wird dann gefriergetrocknet oder bei 120 °C im Trockenschrank von Restfeuchte befreit. Nachdem bei 400 °C die organischen Bestandteile in einem Muffelofen pyrolisiert wurden, wird das Material 2 h bei 1.000 °C kristallisiert. Das so erhaltene Material kann über dem Fachmann bekannte Sieb- oder Tampondruckverfahren als Schicht auf einem Sensorsubstrat (Al₂O₃) abgeschieden oder in kommerzielle Sensorplattformen (z. B. von Heraeus) implementiert werden.

### 2. Beispiel für BaCeO₃ mit Kupferdotierung

8,506 g Ba(O^{t}Bu)₂ (0,03 mol) werden in absolutem PrⁱOH (200 ml) suspendiert, und eine stöchiometrische Menge Ce(OⁱPr)₄ (9,827 g, 0,03 mol) wird langsam unter Rühren unter Schutzgasatmosphäre dazugegeben. Die trübe Mischung wird 6 h bis zum Erhalt einer klaren Lösung gerührt. Zu diesem Zeitpunkt hat sich ein gemischtes Ba-Ce-Alkoxid, genaugenommen BaCe[(O^{t}Bu)₂(OⁱPr)₄], gebildet, das im Gegensatz zu den ursprünglichen Alkoholaten in PrⁱOH löslich ist. Anschließend kann eine Dotierung mit einer Kupferquelle wie CuCl₂ in Alkohol oder einem Cu-Alkoxid in der gewünschten Stöchiometrie erfolgen. Dazu werden 0,255 g CuCl₂ (5 Mol-%) in 10 ml Isopropanol dazugegeben und für 2 h stark gerührt. Man erhält ein transparentes grünes Sol. Das dotierte Ba-Ce-Alkoxidsol wird mit einer stöchiometrischen Menge einer 1,0 molaren Wasserlösung in Isopropanol unter heftigem Rühren vermengt. Das weiterhin klare Sol wird bei 45 °C Badtemperatur am Rotationsverdampfer eingeengt. 110 g eines solchen Cu dotierten hydrolisierten BaCeO₃-Sols (Feststoffgehalt: 10 %) werden 6 h bei 250 °C in einem 250 ml Rührautoklaven mit Tefloninnengefäß bei einem Innendruck von 50 bar autoklaviert. Nach Abkühlen erhält man ein weißes Pulver, das mehrfach mit Alkohol und Wasser gewaschen wird. Röntgendiffraktometrie ergibt phasenreines BaCeO₃ mit geringen Spuren an CuO.

Anstelle von Cu dotiertem BaCeO₃ sind eine Reihe anderer Nanomaterialien als CO₂-Sensormaterial geeignet.
Diese umfassen anstelle von Ce, Elemente der 4., 7., 13. und 14. Gruppe insbesondere Ce, Ti, Zr, In, Sn, Mn sowie Mischungen aus zwei oder mehr Elementen daraus.

Anstelle von Ba, können Elemente der 1., 2. und 3 Gruppe verwendet werden, insbesondere Li, Mg, Ca, Sr, Ba, Y, und La sowie Mischungen aus zwei oder mehr Elementen daraus.

Die sensoren weisen Dotierungen zwischen 0,01 und 20 Atom-%, insbesondere zwischen 0,1 und 10, ganz besonders zwischen 1 und 6 % auf. Die Mischmetalloxide weisen Dotierungen zwischen 1 und 6 Atom-% auf.
Als Dotierion ist neben Cu geeignet: Elemente der Lanthaniden insbesondere: Pr, Nd, Sm, Eu, Gd, La, Er, etc., sowie Mischungen aus zwei oder mehr Elementen daraus sowie Elemente der 3., 10., 11., 12 und 13. Gruppe, insbesondere In, Ga, Zn, Co, Ni, Cu, Ag, Au, Pt, oder Pd sowie Mischungen aus zwei oder mehr Elementen daraus.

In einer anderen besonderen Ausführungsform werden Metall/Metallkompositverbindungen hergestellt, indem metallorganische Precursoren, wie in den zuvor beschriebenen Beispielen zerset werden, jedoch so, daß nicht nur Metalloxide sondern auch elementare Metalle in einer Metalloxidmatrix entstehen können. Diese ternären Komposite sind als gassensitive, insbesondere als CO₂-sensitive Materialien einsetzbar. Dies liegt daran, daß durch die elementaren Metalle zusätzliche Ladungsträger in das System eingebracht werden, was die Leitfähigkeit des halbleitenden Basismaterials so verbessert, daß das gesamte Komposit sehr empfindlich beispielsweise auf CO₂ reagiert. Das Precursormolekül liefert dabei das Templat für eine submikroskopische Vermischung der einzelnen Komponenten.

Im Fall eines Cu/Al₂O₃-Komposits geht man dabei von einem Precursor für CuAl₂O₄ wie beispielsweise Cu[Al₂(OⁱPr)₈] aus. Das Hydrolysat eines solchen Precursors zersetzt sich thermisch unter reduzierenden Bedingungen zu einem homogenen nanoskaligen Cu/Al₂O₃- Komposit. Wird die reduzierende Atmosphäre variiert, entstehen CuO/Al₂O₃ bzw. Cu₂O/ Al₂O₃-Komposite bzw. Gemische aus Cu/Cu₂O/CuO in einer Al₂O₃-Matrix.

Weitere durch die beschriebene oder andere Methoden herstellbare Single-Source-Kompositverbindungen umfassen unter anderem Cu bzw. Cu₂O oder CuO in einer TiO₂-Matrix oder Sn, Cu bzw. Cu₂O oder CuO in einer SnO₂-Matrix.

Als weitere Matrixmaterialien sind ZrO₂, CeO₂, Fe₂O₃, SiO₂, und Y₂O₃ geeignet.

Als Metall bzw. gegebenenfalls Metalloxid in der Matrix sind neben Cu oder Sn Elemente der Lanthaniden insbesondere: Pr, Nd, Sm, Eu, Gd, La, Er, etc., sowie Mischungen aus zwei oder mehr Elementen daraus, sowie Elemente der 3., 8., 9., 10., 11., 12 und 13. Gruppe, insbesondere In, Ga, Zn, Co, Ni, Ru, Os, Rh, Ir, Cu, Ag, Au, Pt, oder Pd sowie Mischungen aus zwei oder mehr Elementen daraus geeignet.

## Patentansprüche

1. Sensor zur Detektion von CO₂, herstellbar durch Single-Source-Precursortechnik, **dadurch gekennzeichnet, dass** er mindestens ein Mischmetalloxid der Formel AXO₃ aufweist, wobei
- A mindestens ein Element ausgewählt aus den Elementen der 1., 2. und 3. Gruppe des Periodensystems und
- X mindestens ein Element ausgewählt aus den Elementen Cer und den Elementen der 4., 7., 13. und 14. Gruppe des Periodensystems ist,
wobei die mittlere Partikelgröße des Mischmetalloxids < 100 nm ist und das Mischmetalloxid dotiert ist, wobei der Gehalt an Dotierelementen zwischen 0,01 und 20 Atom-% beträgt.

2. Sensor nach Anspruch 1, **dadurch gekennzeichnet, dass** die mittlere Partikelgröße des Mischmetalloxids < 50 nm ist.

3. Sensor nach einem der Ansprüche 1 oder 2, **dadurch** gekenneichnet, dass zur Dotierung mindestens ein Element ausgewählt aus den Elementen der 3., 10., 11., 12. und 13. Gruppe des Periodensystems und der Lanthanoide vorhanden ist.

4. Sensor nach Anspruch 3, **dadurch gekennzeichnet, dass** es sich bei dem Dotierelement um Kupfer handelt.

5. Sensor nach einem der vorhergehenden Ansprüche, **dadurch gekennzeichnet, dass** der Gehalt an Dotierelementen zwischen 0,1 und 10 Atom-%, insbesondere zwischen 1 und 6 Atom-%, beträgt.

6. Sensor nach einem der vorhergehenden Ansprüche, **dadurch gekennzeichnet, dass** A aus den Elementen der 2. Gruppe des Periodensystems ausgewählt ist, wobei es sich vorzugsweise um Barium handelt.

7. Sensor nach einem der vorhergehenden Ansprüche, **dadurch gekennzeichnet, dass** X Cer ist.

8. Sensor nach einem der vorhergehenden Ansprüche, **dadurch gekennzeichnet, dass** X Titan oder Zirkonium ist.

9. Sensor nach einem der vorhergehenden Ansprüche, **dadurch gekennzeichnet, dass** das Mischmetalloxid die Formel BaXO₃ hat, wobei X Cer ist.

10. Sensor nach Anspruch 9, **dadurch gekennzeichnet, dass** das Mischmetalloxid mit Kupfer dotiert ist.

11. Mischmetalloxid der Formel AXO₃, herstellbar durch Single-Source-Precursortechnik, wobei
- A mindestens ein Element ausgewählt aus den Elementen der 1., 2. und 3. Gruppe des Periodensystems und
- X mindestens ein Element ausgewählt aus den Elementen Cer und den Elementen der 4., 7., 13. und 14. Gruppe des Periodensystems ist,
wobei die mittlere Partikelgröße des Mischmetalloxids < 100 nm ist und das Mischmetalloxid dotiert ist, wobei der Gehalt an Dotierelementen zwischen 1 und 6 Atom-% beträgt, und Mischungen solcher Mischmetalloxide.

12. Mischmetalloxid nach Anspruch 11, **dadurch gekennzeichnet, dass** die mittlere Partikelgröße < 50 nm ist.

13. Mischmetalloxid nach einem der Ansprüche 11 oder 12, **dadurch gekennzeichnet, dass** zur Dotierung mindestens ein Element ausgewählt aus den Elementen der 3., 10., 11., 12. und 13. Gruppe des Periodensystems und der Lanthanoide vorhanden ist.

14. Mischmetalloxid nach Anspruch 13, **dadurch gekennzeichnet, dass** es sich bei dem Dotierelement um Kupfer handelt.

15. Mischmetalloxid nach einem der Ansprüche 11 bis 14, **dadurch gekennzeichnet, dass** A aus den Elementen der 2. Gruppe des Periodensystems ausgewählt ist, wobei es sich vorzugsweise um Barium handelt.

16. Mischmetalloxid nach einem der Ansprüche 11 bis 15, **dadurch gekennzeichnet, dass** X Cer ist.

17. Mischmetalloxid nach einem der Ansprüche 11 bis 16, **dadurch gekennzeichnet, dass** X Titan oder Zirkonium ist.

18. Mischmetalloxid nach einem der Ansprüche 11 bis 17, **dadurch gekennzeichnet, dass** das Mischmetalloxid die Formel BaXO₃ hat, wobei X Cer ist.

19. Mischmetalloxid nach Anspruch 18, **dadurch gekennzeichnet, dass** das Mischmetalloxid mit Kupfer dotiert ist.

20. Mischmetalloxid nach einem der Ansprüche 11 bis 19, **dadurch gekennzeichnet, dass** es auf ein Substrat aufgebracht und/oder in ein Substrat eingebracht ist.

21. Mischmetalloxid nach Anspruch 20, **dadurch gekennzeichnet, dass** es sich bei dem Substrat um ein Substrat für Sensoren, insbesondere für Gassensoren handelt.

22. Verwendung der Mischmetalloxide nach einem der Ansprüche 11 bis 21 zur Detektion von Gasen, vorzugsweise zur Detektion nicht brennbarer Gase.

23. Verfahren zur Herstellung von Mischmetalloxiden nach einem der Ansprüche 11 bis 22, **dadurch gekennzeichnet, dass** mit Hilfe der sogenannten Single-Source-Precursortechnik ein Mischmetallalkoxid hergestellt wird, das in der Stöchiometrie und der Struktur auf das herzustellende Mischmetalloxid abgestellt ist, und dieses Mischmetalloxid nach einem Dotierungsschritt zu dem Mischmetalloxid hydrolysiert wird.

## Claims

1. A sensor for the detection of CO₂, preparable by a single-source precursor technology, **characterized in that** it includes at least one mixed metal oxide of the formula AXO₃, where
- A is at least one element selected from the elements of group 1, 2 and 3 of the periodic table, and
- X is at least one element selected from the elements cerium and the elements of group 4, 7, 13 and 14 of the periodic table,
wherein the average particle size of the mixed metal oxide is < 100 nm, and the mixed metal oxide is doped, wherein the content of doping elements is between 0.01 and 20 atomic %.

2. The sensor according to claim 1, **characterized in that** the average particle size of the mixed metal oxide is < 50 nm.

3. The sensor according to claim 1 or 2, **characterized in that** at least one element selected from the elements of group 3, 10, 11, 12 and 13 of the periodic table and the lanthanoids is present for doping.

4. The sensor according to claim 3, **characterized in that** the doping element is copper.

5. The sensor according to any of the preceding claims, **characterized in that** the content of doping elements is between 0.1 and 10 atomic %, in particular between 1 and 6 atomic %.

6. The sensor according to any of the preceding claims, **characterized in that** A is selected from the elements of group 2 of the periodic table, and is preferably barium.

7. The sensor according to any of the preceding claims, **characterized in that** X is cerium.

8. The sensor according to any of the preceding claims, **characterized in that** X is titanium or zirconium.

9. The sensor according to any of the preceding claims, **characterized in that** the mixed metal oxide has the formula BaXO₃, where X is cerium.

10. The sensor according to claim 9, **characterized in that** the mixed metal oxide is doped with copper.

11. A mixed metal oxide of the formula AXO₃, preparable by a single-source precursor technology, wherein
- A is at least one element selected from the elements of group 1, 2 and 3 of the periodic table, and
- X is at least one element selected from the elements cerium and the elements of group 4, 7, 13 and 14 of the periodic table,
wherein the average particle size of the mixed metal oxide is < 100 nm, and the mixed metal oxide is doped, wherein the content of doping elements is between 1 and 6 atomic %, and mixtures of these mixed metal oxides.

12. The mixed metal oxide according to claim 11, **characterized in that** the average particle size is < 50 nm.

13. The mixed metal oxide according to claim 11 or 12, **characterized in that** at least one element selected from the elements of group 3, 10, 11, 12 and 13 of the periodic table and the lanthanoids is present for doping.

14. The mixed metal oxide according to claim 13, **characterized in that** the doping element is copper.

15. The mixed metal oxide according to any of the claims 11 to 14, **characterized in that** A is selected from the elements of group 2 of the periodic table, and is preferably barium.

16. The mixed metal oxide according to any of the claims 11 to 15, **characterized in that** X is cerium.

17. The mixed metal oxide according to any of the claims 11 to 16, **characterized in that** X is titanium or zirconium.

18. The mixed metal oxide according to any of the claims 11 to 17, **characterized in that** the mixed metal oxide has the formula BaXO₃, where X is cerium.

19. The mixed metal oxide according to claim 18, **characterized in that** the mixed metal oxide is doped with copper.

20. The mixed metal oxide according to any of the claims 11 to 19, **characterized in that** it is applied to a substrate and/or incorporated into a substrate.

21. The mixed metal oxide according to claim 20, **characterized in that** the substrate is a substrate for sensors, in particular for gas sensors.

22. Use of the mixed metal oxides according to any of the claims 11 to 21 for detecting gases, preferably for detecting incombustible gases.

23. A process for preparing mixed metal oxides according to any of the claims 11 to 21, **characterized in that** using the so-called single-source precursor technique a mixed metal alkoxide is prepared, whose stoichiometry and structure are adjusted to the mixed metal oxide to be prepared, and this mixed metal alkoxide, after a doping step, is hydrolyzed to the mixed metal oxide.

## Revendications

1. Sonde de détection de CO₂ fabriquée par une technique de précurseur à source unique,
**caractérisée en ce que**
elle présente au moins un oxyde métallique mixte de formule AXO₃ dans laquelle
- A représente au moins un élément sélectionné parmi les éléments des groupes 1, 2 et 3 du système périodique et
- X représente au moins un élément sélectionné parmi l'élément cérium et les éléments des groupes 4, 7, 13 et 14 du système périodique,
**en ce que** la taille moyenne des particules d'oxyde métallique mixte est < 100 nm,
**en ce que** l'oxyde métallique mixte est dopé et
**en ce que** la teneur en élément dopant est comprise entre 0,01 et 20 % atomiques.

2. Sonde selon la revendication 1, **caractérisée en ce que** la taille moyenne des particules de l'oxyde métallique mixte est < 50 nm.

3. Sonde selon l'une des revendications 1 ou 2, **caractérisée en ce que** pour le dopage, elle présente au moins un élément sélectionné parmi les éléments des groupes 3, 10, 11, 12 et 13 du système périodique et les lanthanoïdes.

4. Sonde selon la revendication 3, **caractérisée en ce que** l'élément dopant est le cuivre.

5. Sonde selon l'une des revendications précédentes, **caractérisée en ce que** la teneur en élément dopant est comprise entre 0,1 et 10 % atomiques et en particulier entre 1 et 6 % atomiques.

6. Sonde selon l'une des revendications précédentes, **caractérisée en ce que** A est sélectionné parmi les éléments du groupe 2 du système périodique et est de préférence le baryum.

7. Sonde selon l'une des revendications précédentes, **caractérisée en ce que** X représente le cérium.

8. Sonde selon l'une des revendications précédentes, **caractérisée en ce que** X représente le titane ou le zirconium.

9. Sonde selon l'une des revendications précédentes, **caractérisée en ce que** l'oxyde métallique mixte a la formule BaXO₃ dans laquelle X représente le cérium.

10. Sonde selon la revendication 9, **caractérisée en ce que** l'oxyde métallique mixte est dopé au cuivre.

11. Oxyde métallique mixte de formule AXO₃ fabriqué par une technique de précurseur à source unique, et dans lequel
- A représente au moins un élément sélectionné parmi les éléments des groupes 1, 2 et 3 du système périodique et
- X représente au moins un élément sélectionné parmi l'élément cérium et les éléments des groupes 4, 7, 13 et 14 du système périodique,
la taille moyenne des particules d'oxyde métallique mixte étant < 100 nm,
l'oxyde métallique mixte étant dopé et
la teneur en élément dopant étant comprise entre 1 et 6 % atomiques,
ainsi que des mélanges de ces oxydes métalliques mixtes.

12. Oxyde métallique mixte selon la revendication 11, **caractérisé en ce que** la taille moyenne des particules est < 50 nm.

13. Oxyde métallique mixte selon l'une des revendications 11 ou 12, **caractérisé en ce que** pour le dopage, il présente au moins un élément sélectionné parmi les éléments des groupes 3, 10, 11, 12 et 13 du système périodique et les lanthanoïdes.

14. Oxyde métallique mixte selon la revendication 13, **caractérisé en ce que** l'élément dopant est le cuivre.

15. Oxyde métallique mixte selon l'une des revendications 11 à 14, **caractérisé en ce que** A est sélectionné parmi les éléments du groupe 2 du système périodique et est de préférence le baryum.

16. Oxyde métallique mixte selon l'une des revendications 11 à 15, **caractérisé en ce que** X représente le cérium.

17. Oxyde métallique mixte selon l'une des revendications 11 à 16, **caractérisé en ce que** X représente le titane ou le zirconium.

18. Oxyde métallique mixte selon l'une des revendications 11 à 17, **caractérisé en ce que** l'oxyde métallique mixte a la formule BaXO₃ dans laquelle X représente le cérium.

19. Oxyde métallique mixte selon la revendication 18, **caractérisé en ce que** l'oxyde métallique mixte est dopé au cuivre.

20. Oxyde métallique mixte selon l'une des revendications 11 à 19, **caractérisé en ce qu'**il est appliqué sur un substrat et/ou incorporé dans un substrat.

21. Oxyde métallique mixte selon la revendication 20, **caractérisé en ce que** le substrat est un substrat de sonde et en particulier de sonde de gaz.

22. Utilisation des oxydes métalliques mixtes selon l'une des revendications 11 à 21 pour la détection de gaz et de préférence pour la détection de gaz non combustibles.

23. Procédé de fabrication d'oxydes métalliques mixtes selon l'une des revendications 11 à 22, **caractérisé en ce qu'**à l'aide de la technique dite de précurseur à source unique, on prépare un alkoxyde métallique mixte dont la stoechiométrie et la structure correspondent à celles de l'oxyde métallique mixte à préparer et on hydrolyse cet alkoxyde métallique mixte en l'oxyde métallique mixte après une étape de dopage.
